(19) Europäisches Patentamt
European Patent Office
Office européen des brevets

(11) Veröffentlichungsnummer: **0 220 137 B1**

(12) # EUROPÄISCHE PATENTSCHRIFT

(45) Veröffentlichungstag der Patentschrift: **19.06.91**

(51) Int. Cl.$^5$: **C07D 209/76**, C07F 9/09, C07F 9/145, C07F 9/53, C08F 26/06, C08F 30/02, C08F 22/40

(21) Anmeldenummer: **86810465.4**

(22) Anmeldetag: **20.10.86**

Die Akte enthält technische Angaben, die nach dem Eingang der Anmeldung eingereicht wurden und die nicht in dieser Patentschrift enthalten sind.

(54) **Trisimide von allyl- oder methallyl-substituierten Bicyclo[2.2.1]hept-5-en-2,3-dicarbonsäureimiden und deren Verwendung.**

(30) Priorität: **25.10.85 CH 4610/85**

(43) Veröffentlichungstag der Anmeldung:
**29.04.87 Patentblatt 87/18**

(45) Bekanntmachung des Hinweises auf die Patenterteilung:
**19.06.91 Patentblatt 91/25**

(84) Benannte Vertragsstaaten:
**CH DE FR GB IT LI NL**

(56) Entgegenhaltungen:
**EP-A- 0 105 024**
**EP-A- 0 134 041**
**EP-A- 0 146 498**
**EP-A- 0 152 372**
**EP-A- 0 155 435**
**DE-A- 2 350 472**

(73) Patentinhaber: **CIBA-GEIGY AG**
**Klybeckstrasse 141**
**CH-4002 Basel(CH)**

(72) Erfinder: **Vonlanthen, Christian**
**La Verdure**
**CH-1711 Ependes(CH)**
Erfinder: **Kramer, Andreas, Dr.**
**Bundtels**
**CH-3186 Düdingen(CH)**
Erfinder: **Renner, Alfred, Dr.**
**Marcoup 2**
**CH-3286 Muntelier(CH)**

**EP 0 220 137 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Trisimide von allyl- oder methallyl-substituierten Bicyclo[2.2.1]-hept-5-en-2,3-dicarbonsäureimiden, ein Verfahren zu deren Herstellung und deren Verwendung zur Herstellung von Polymeren durch Erwärmen.

In den europäischen Patentveröffentlichungen 0 105 024 A1 und 0 152 372 A2 sind allyl- oder methallyl-substituierte [Methyl]Bicyclo[2.2.1]hept-5-en-2,3-dicarbonsäureimide beschrieben, aus denen durch Erhitzen vernetzte Polymere hergestellt werden können. Aus der DE-OS 23 50 472 sind ferner ungesättigte Trisimide, vor allem Tris-maleinimide, von aromatischen Aminophosphaten und -phosphiten bekannt.

Gegenstand der Erfindung sind Verbindungen der Formel I

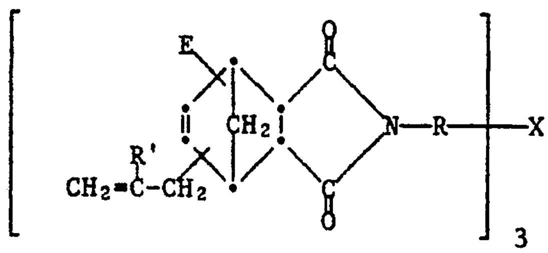

(I),

worin E und R' unabhängig voneinander ein Wasserstoffatom oder Methyl bedeuten,

X 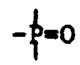 oder 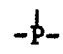

und R $-C_mH_{2m}-$ mit m = 2-20, $-C_nH_{2n}-O-$mit n = 2-6, meta- oder para-Phenylen, meta- oder para-Phenylenoxy darstellen, wobei das Sauerstoffatom an die Gruppe X gebunden ist, oder

X 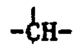

und R meta- oder para-Phenylen darstellen, oder X $CH_3CH_2C(CH_2-)_3$ oder

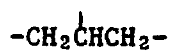

ist und R $-(CH_2CH_2O)_q-$, $-(CH_2CH_2CH_2O)_q-$ oder $-[CH_2CH(CH_3)O]_q-$ mit q = 1-6, meta- oder para-Phenylenoxy darstellt, wobei das Sauerstoffatom an die Gruppe X gebunden ist, oder

X 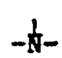

und R $-C_rH_{2r}-$ mit r = 2-4, meta- oder para-Phenylen darstellen.

Stellt R $-C_mH_{2m}-$ oder $-C_nH_{2n}-O-$dar, so kann es sich um geradkettige oder verzweigte Alkylen- bzw. Alkylenoxygruppen handeln. Als Beispiele seien genannt: 1,2-Ethylen, 1,3- und 1,2-Propylen, Tetramethylen, Pentamethylen, 2,2-Dimethyl-1,3-propylen, Hexamethylen, Heptamethylen, Octamethylen, Decamethylen, Dodecamethylen, Tetradecamethylen, Pentadecamethylen, Hexadecamethylen, Octadecamethylen und Eicosylen; Ethylenoxy, n-Propylenoxy, 1,2-Propylenoxy, n-Butylenoxy, n-Pentylenoxy oder n-Hexylenoxy. Bevorzugt sind geradkettige Gruppen $-C_mH_{2m}-$ oder $-C_nH_{2n}-O-$ mit m = 2-6 und besonders 2-4 C-Atomen und n = 2-4 C-Atomen.

Bedeutet R $-(CH_2CH_2O)_q-$, $-(CH_2CH_2CH_2O)_q-$ oder $-(CH_2CH(CH_3)O)_q-$, so können die q in den drei R unterschiedliche Bedeutung haben. Bevorzugt liegen die Werte von q dabei durchschnittlich zwischen 1,2 und 3, besonders 1,2 und 2.

Ist R eine Gruppe $-C_rH_{2r}-$, so kann es sich um geradkettige oder verzweigte Reste handeln, wie 1,2-Ethylen, 1,3- oder 1,2-Propylen und 1,4-Butylen. Bevorzugt sind Gruppen $-(CH_2)_r-$ mit r = 2 oder 3.

E und R' stellen bevorzugt je ein Wasserstoffatom dar. X ist vorzugsweise

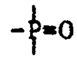 oder 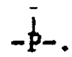.

Besonders bevorzugt sind Verbindungen der Formel I, worin

X 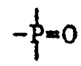

oder 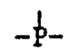

darstellt und R $-(CH_2)_m-$ mit m = 2-4, meta- oder para-Phenylen, meta- oder para-Phenylenoxy, insbesondere para-Phenylenoxy, bedeutet.

Stellt

X 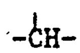

dar, so bedeutet R bevorzugt p-Phenylen. Eine weitere Klasse bevorzugter Verbindungen der Formel I sind solche, worin X $CH_3CH_2C(CH_2-)_3$ ist und die R $-[CH_2CH(CH_3)O]_q-$ mit q = durchschnittlich 1,2 - 3, besonders 1,2 - 2, darstellen, sowie Verbindungen der Formel I, worin

X 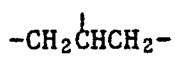

ist und die R $-(CH_2CH_2O)_q-$ mit q = durchschnittlich 1,2-3, besonders 1,2-2 und vor allem meta-oder para-Phenylenoxy bedeuten. Stellt

X 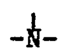

dar, so bedeutet R bevorzugt $-(CH_2)_r-$ mit r = 2 oder 3, meta- oder para-Phenylen.

Die Verbindungen der Formel I können auf an sich bekannte Weise hergestellt werden, indem man eine Verbindung der Formel II

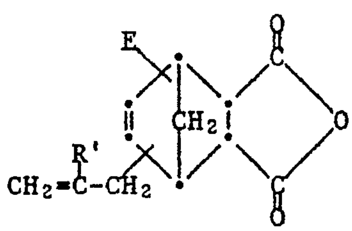

(II)

bei erhöhter Temperatur und unter Abdestillieren des bei der Reaktion entstehenden Wassers mit einer Verbindung der Formel III

$[H_2N-R]_3-X$ (III)

umsetzt, wobei für E, R, R' und X das oben Angegebene gilt.

Die Umsetzung kann ohne Lösungsmittel oder in Gegenwart eines inerten Lösungsmittels, das für die azeotrope Entfernung des Wassers verwendbar ist (Schleppmittel, wie Toluol und Xylole) erfolgen. Die Temperaturen für die Umsetzung in Gegenwart eines Lösungsmittels können zwischen 100°C und

Rückflusstemperatur liegen. Die Umsetzung in der Schmelze erfolgt zweckmässig bei Atmosphärendruck bei Temperaturen zwischen 100 und 250 °C, insbesondere 130 und 220 °C. Die Umsetzung in Gegenwart eines inerten Lösungsmittels ist bevorzugt.

Verbindungen der Formel I, worin

$$-\overset{|}{\underset{|}{P}}=O \text{ oder } -\overset{|}{P}-$$

und R $-C_nH_{2n}$, meta- oder para-Phenylen darstellen, können nach einem Verfahren auch dadurch erhalten werden, dass man eine Verbindung der Formel IV

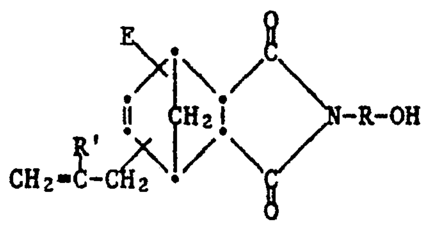 (IV),

worin E und R' die unter Formel I angegebene Bedeutung haben und R $-C_nH_{2n}$, meta- oder para-Phenylen darstellt, in Gegenwart einer Base im Molverhältnis von mindestens 1:3 mit einem Phosphoroxyhalogenid oder einem Phosphortrihalogenid, besonders Phosphoroxychlorid, Phosphortribromid oder Phosphortrichlorid, umsetzt.

Als Basen eignen sich z.B. tertiäre Amine, wie Triethylamin, Tri-n-butylamin, Pyridin und Dimethylanilin. Diese Umsetzung wird zweckmässig in einem inerten organischen Lösungsmittel, wie Toluol oder Xylolen, bei Temperaturen zwischen -50 °C und +50 °C, besonders 0-30 °C, durchgeführt.

Stellt

$$X\ -\overset{|}{\underset{|}{P}}=O \text{ oder } -\overset{|}{P}-$$

dar, so kann es unter Umständen zweckmässig sein, bei den obigen Reaktionen in Gegenwart eines inerten Lösungsmittels ein Antioxidans mitzuverwenden.

Die Verbindungen der Formeln II, III und IV sind bekannt oder können nach an sich bekannten Methoden hergestellt werden. Verbindungen der Formel II können z.B. nach dem in der US-PS 3.105.839 beschriebenen Verfahren erhalten werden, indem man Natrium-Cyclopentadienid oder Natrium-Methylcyclopentadienid mit einem Allyl- oder Methallylhalogenid umsetzt, worauf sich eine Diels-Alder-Reaktion mit Maleinsäureanhydrid anschliesst. Obgleich in der US Patentschrift angegeben wird, dass die Allylgruppe in 7-Stellung des bicyclischen Systems gebunden ist, zeigen neuere Untersuchungen, dass eine isomere Mischung in Bezug auf die Stellung der Allyl- bzw. Methallylgruppe (in 1- und 6-Position) und auch auf die Endo- und Exokonfiguration des Anhydridteils gebildet wird. Verbindungen der Formel IV können durch Umsetzung eines Anhydrids der Formel II mit den entsprechenden Aminoalkoholen $H_2N$-R-OH hergestellt werden, wobei diese Umsetzung ebenfalls bei erhöhter Temperatur und unter Abdestillieren des bei der Reaktion entstehenden Wassers vorgenommen wird. Verbindungen der Formel III mit

$$X = -\overset{|}{\underset{|}{P}}=O \text{ oder } -\overset{|}{P}-$$

können z.B. durch Umsetzung von Aminoalkoholen $H_2N$-R-OH mit Phosphoroxyhalogeniden oder Phosphortrihalogeniden erhalten werden.

Die Verbindungen der Formel I fallen im allgemeinen in Form fester Harze an, die zu vernetzten Produkten mit hohen Glasumwandlungstemperaturen polymerisiert werden können. Sie können vielseitig angewendet werden, z.B. als hitzebeständige Klebstoffe, als Isoliermaterialien in der Elektronik und Elektrotechnik und vor allem als Matrixharze für Verbundwerkstoffe. Die Trisimide der Formel I können allein oder zusammen mit anderen ungesättigten Imiden, z.B. solchen der in den europäischen Patentveröffentlichungen 0 105 024 A1 und 0 152 372 A2 beschriebenen Art, polymerisiert werden. Die Polymerisation wird zweckmässig bei Temperaturen zwischen 180 und 300 °C, vorzugsweise 200-260 °C, durchgeführt. Die Reaktionszeiten liegen im allgemeinen zwischen 6 und 50, besonders 6 und 20 Stunden. In manchen Fällen kann es auch vorteilhaft sein, die Polymerisation in zwei Stufen vorzunehmen, indem man zuerst bei relativ niedrigen Temperaturen, z.B. zwischen 180 und 220 °C, eine Vorgelierung durchführt und anschliessend bei erhöhten Temperaturen, z.B. zwischen 240 und 300 °C, auspolymerisiert.

Gegenstand der Erfindung sind daher auch Polymere, die dadurch erhältlich sind, dass man mindestens ein Trisimid der Formel I auf Temperaturen zwischen 180 und 300 °C erhitzt.

Die Trisimide der Formel I zeichnen sich durch eine hohe Reaktivität aus. Trotz ihrer hohen Reaktivität sind sie jedoch im allgemeinen noch schmelz- bzw. giessbar. Die aus den Verbindungen der Formel I hergestellten Polymeren weisen wertvolle Eigenschaften, besonders gute mechanische und vor allem gute thermische Beständigkeit, wie hohe Glasumwandlungs- und Zersetzungstemperaturen und geringen Gewichtsverlust bei erhöhten Temperaturen, auf. Polymere aus Trisimiden der Formel I, worin

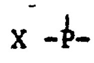 oder 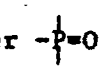

darstellt, finden ferner Anwendung als selbstlöschende Kunststoffe.

Beispiel 1:

Herstellung des N,N',N"-Tris-(allyl-bicyclo[2.2.1]hept-5-en-2,3-dicarbonsäureimids) des Tris-(4-aminophenyl)phosphats. 204 g Allyl-bicyclo[2.2.1]hept-5- -en-2,3-dicarbonsäureanhydrid (Allylnadic-anhydrid, hergestellt gemäss US-PS 3.105.839) 109,1 g 4-Aminophenol, 0,16 g der Verbindung der Formel

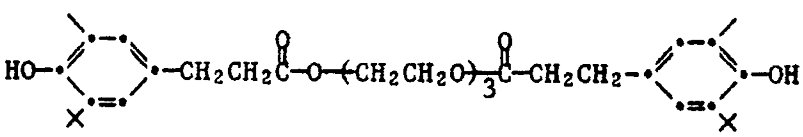 (Antioxidans Nr.1)

werden in 500 g eines Xylol-Isomerengemisches zum Sieden erhitzt. Das bei der Reaktion entstehende Wasser wird mittels eines Hahn'schen Aufsatzes abgetrennt. Nach 5 Stunden bei 120-123 °C ist die Reaktion beendet. Man klärt durch Filtration (über einem Filtriergerät Hyflo-Supercel). setzt 111,3 g Triethylamin zu, kühlt auf 0 °C und tropft im Verlaufe einer Stunde unter Rühren und äusserer Kühlung 51 g Phosphoroxychlorid zu. Man rührt über Nacht bei Raumteperatur, stellt den pH-Wert mit 50 ml n-HCl auf 6, löst das ausgeschiedene Triethylaminohydrochlorid in 400 ml Wasser, wäscht die Xylollösung zweimal mit je 600 ml Wasser, trocknet über $Na_2SO_4$, engt am Rotationsverdampfer ein und hält 1 Stunde bei 1700 Pa und 150 °C. Man erhält 263,5 g (85 % d.Th.) des N,N',N"-Tris-(allyl-bicyclo[2.2.1]hept-5-en-2,3-dicarbonsäureimids) des Tris-(4-aminophenyl)phosphats in Form eines rotbraunen festen Harzes mit einer Glasumwandlungstemperatur von 66,5 °C.

**Elementaranalyse für $C_{54}H_{48}N_3O_{10}P$:**

| | | | | |
|---|---|---|---|---|
| berechnet | C 69,74 % | H 5,20 % | N 4,52 % | P 3,33 % |
| gefunden | C 69,31 % | H 5,39 % | N 4,22 % | P 3,45 %. |

IR-Spektrum: 1201 $cm^{-1}$ Phosphonylgruppe, 1620 $cm^{-1}$ cyclische Doppelbindung, 1640 $cm^{-1}$ Allylgruppe, 1710 $cm^{-1}$ und 1776 $cm^{-1}$ für die Carbonylgruppe.

Beispiel 2:

61,2 g Allylnadic-anhydrid und 37,1 g Tris-(4-aminophenyl)-phosphat [Fp. 152-155 °C, hergestellt gemäss US-PS 3.415.779, Produkt I) werden gemischt und unter Rühren bei 1,06 Pa auf 155 °C erhitzt. Man giesst das erhaltene rotbraune Harz auf eine Blechtasse aus. Das Harz (Verbindung identisch mit derjenigen von Beispiel 1) erstarrt sofort; es weist eine Glasumwandlungstemperatur von 67 °C auf. Ausbeute 93 g (quantitativ).

**Elementaranalyse für $C_{54}H_{48}N_3O_{10}P$:**

| | | | | | |
|---|---|---|---|---|---|
| **berechnet** | C 69,74 % | H 5,20 % | N 4,52 % | P 3,33 % | freies Amin 0 % |
| **gefunden** | C 69,41 % | H 5,20 % | N 4,49 % | P 3,37 % | freies Amin 0,07 %. |

Das IR-Spektrum stimmt mit dem Spektrum der gemäss Beispiel 1 erhaltenen Verbindung überein.

Beispiel 3:

Herstellung des N,N', N"-Tris(allyl-bicyclo[2.2.1]hept-5-en-2,3-dicarbonsäureimids) des Tris-(3-aminophenyl)phosphits. Allyl-bicyclo[2.2.1]hept-5-en-2,3-dicarbonsäure-N-(3'-hydroxyphenyl)imid wird durch azeotrope Entwässerung eines Reaktionsgemisches aus 306 g Allylnadic-anhydrid, 163,7 g 3-Aminophenol und 750 g Xylol hergestellt. Nach dem Abdestillieren des Lösungsmittels verbleiben 330,4 g eines rotbraunen Festharzes mit einer Glasumwandlungstemperatur (Tg) von 60 °C. Man löst 324,5 g dieses Zwischenprodukts in 750 g Toluol, setzt 122,45 g Triethylamin zu und tropft unter Rühren und äusserer Kühlung 50,3 g Phosphortrichlorid so zu, dass die Temperatur zwischen 7 und 11 °C verbleibt. Man rührt über Nacht bei Raumtemperatur, neutralisiert mit 1n-HCl, wäscht zweimal mit Wasser, trocknet die Toluolphase über $Na_2SO_4$, filtriert und engt am Rotationsverdampfer ein (150 °C/1600 Pa). Man erhält 219,4 g (72 % d.Th.) N, N', N"-Tris(allyl-bicyclo[2.2.1]hept-5-en-2,3-dicarbonsäureimid) des Tris(3-aminophenyl)phosphits in Form eines dunklen Festharzes; Tg = 55 °C.

**Elementaranalyse für $C_{54}H_{48}N_3O_9P$:**

| | | | | |
|---|---|---|---|---|
| **berechnet** | C 70,96 % | H 5,29 % | N 4,60 % | P 3,39 % |
| **gefunden** | C 71,42 % | H 5,70 % | N 4,27 % | P 3,30 %. |

Beispiel 4:

Herstellung des N,N' ,N"-Tris(allyl-bicyclo[2.2.1]hept-5-en-2, dicarbonsäureimids) des Tris(3-aminophenyl)phosphinoxids. Man erhitzt eine Lösung von 55,1 g Allylnadic-anhydrid, 29 g Tris(3-aminophenyl)-phosphinoxid (Fp. 251-4 °C) und 42 mg des Antioxidans Nr. 1 in 159 g Xylol zum Sieden und scheidet das bei der Imidisierung entstehende Wasser über ein Dean-Stark Trennrohr ab. Danach leitet man das Destillat vor der Rückführung während 3 Stunden über gekörntes Calciumchlorid. Man kühlt ab, klärt durch Filtration und destilliert das Xylol im Vakuum am Rotationsverdampfer bei 140 °C ab. Es verbleiben 78,0 g (98,4 d.Th.) N,N' ,N"-Tris(allyl-bicyclo[2.2.1]hept-5-en-2,3-dicarbonsäureimid) mit einer Glasumwandlungstemperatur von 95,5 °C.

**Elementaranalyse für $C_{54}H_{48}O_7N_3P$:**

| | | | | |
|---|---|---|---|---|
| **berechnet** | C 73,54 % | H 5,49 % | N 4,76 % | P 3,51 % |
| **gefunden** | C 71,69 % | H 5,75 % | N 4,65 % | P 3,31 %. |

Beispiel 5:

Herstellung des N,N' ,N"-Tris(allyl-bicyclo[2.2.1]hept-5-en-2,3-dicarbonsäureimids) eines Polyoxypropylentriamins. Man erhitzt 61,2 g Allylnadic-anhydrid und 45,8 g eines Polyoxypropylentriamins mit einem durchschnittlichen Molekulargewicht von 403 der Formel

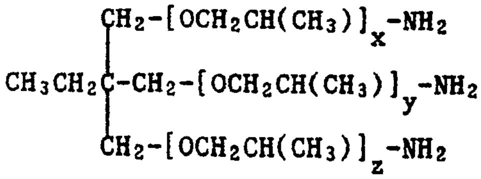

, $x + y + z = 5,3$

(Jeffamine T 403® der Fa. Texaco) auf 200 °C, erniedrigt den Druck auf 6,7 Pa und hält diese Bedingungen während 5 Stunden. Man erhält das N,N' ,N"-Tris(allyl-bicyclo[2.2.1]hept-5-en-dicarbonsäureimid) des Polyoxypropylentriamins als hellbraunes Harz in quantitativer Ausbeute. Das Harz weist bei 120 °C eine Viskosität von 1,817 Pa•s auf.

Beispiel 6:

Herstellung des N,N' ,N"-Tris-(allyl-bicyclo[2.2.1]hept-5-en-2,3-dicarbonsäureimids) des Tris(2-aminoethyl)amins. 21,0 g Allylnadic-anhydrid und 5,0 g Tris(2-aminoethyl)amin werden in 200 ml Toluol vorgelegt und 5 Stunden am Wasserabscheider unter Rückfluss erhitzt. Das Toluol wird im Vakuum am Rotationsverdampfer bei 60 °C abdestilliert, und der Rückstand wird bei 130 °C am Hochvakuum getrocknet. Man erhält 23,5 g (98 % d.Th.) des N,N' ,N"-Tris(allyl-bicyclo[2.2.1]hept-5-en-dicarbonsäureimids) des Tris(2-aminoethyl)amins in Form eines braunroten, bei Raumtemperatur festen Harzes mit einer Glasumwandlungstemperatur von 83,5 °C.

Elementaranalyse für $C_{42}H_{48}N_4O_6$:

| | | | |
|---|---|---|---|
| berechnet | C 71,57 % | H 6,86 % | N 7,95 % |
| gefunden | C 70,82 % | H 6,77 % | N 7,76 %. |

Infrarotspektrum:
1640 cm$^{-1}$ Allylgruppe, 1700 cm$^{-1}$ Carbonylgruppe.

Durch 12-stündige Polymerisation der obigen Verbindung bei 250 °C erhält man einen Festkörper mit einer Glasumwandlungstemperatur über 300 °C.

Beispiel 7:

Herstellung des N,N' ,N"-Tris(Allyl-bicyclo[2.2.1]hept-5-en-2,3-dicarbonsäureimids) des Tris(4-aminophenyl)amins.

Man erhitzt eine Suspension von 43,7 g Allylnadic-anhydrid und 21,6 g Tris(4-aminophenyl)amin in 400 ml Toluol auf 100 °C. Dabei löst sich das Trisamin nur teilweise. Nach der Zugabe von 20 ml N,N-Dimethylformamid erhält man eine klare rotbraune Reaktionslösung, die nun während 4,5 Stunden am Wasserabscheider gekocht wird. Das Lösungsmittel wird anschliessend am Rotationsverdampfer abdestilliert, und der Rückstand wird am Hochvakuum bei 130 °C getrocknet. Man erhält 590,0 g des N,N' ,N"-Tris(allyl-bicyclo[2.2.1]hept-5-en-dicarbonsäureimids) des Tris(4-aminophenyl)amins in Form eines braunen Festharzes mit einer Glasumwandlungstemperatur von 67 °C und einem Schmelzintervall von 155-170 °C.

Elementaranalyse für $C_{54}H_{48}N_4O_6$:

| | | | |
|---|---|---|---|
| berechnet | C 76,40 % | H 5,70 % | N 6,60 % |
| gefunden | C 74,80 % | H 5,9 % | N 6,9 %. |

Infrarotspektrum:
1640 cm$^{-1}$ Allylgruppe, 1720 cm$^{-1}$ Carbonylgruppe.

Durch Polymerisation der obigen Verbindungen während 12 Stunden bei 250 °C erhält man einen Festkörper mit einem Tg über 300 °C.

Beispiel 8:

Herstellung des N,N'N"-Tris(allyl-bicyclo[2.2.1]hept-5-en-2,3-dicarbonsäureimids) des 1,2,3-Tris(4-aminophenoxy)propans. Nach der in Beispiel 6 beschriebenen Arbeitsweise werden 52,8 g Allylnadic-anhydrid und 31,5 g 1,2,3-Tris(4-aminophenyl)propan in 400 ml Toluol umgesetzt. Man erhält 74,9 g (94 % d.Th.) des obigen Trisimids in Form eines rotbraunen Festharzes mit einer Glasumwandlungstemperatur von 94 °C und einem Schmelzintervall von 130-160 °C.

| Elementaranalyse für $C_{57}H_{56}O_9N_3$: | | | |
|---|---|---|---|
| berechnet | C 73,86 % | H 6,04 % | N 4,53 % |
| gefunden | C 73,10 % | H 5,82 % | N 4,42 %. |

Infrarotspektrum:
1710 $cm^{-1}$ Carbonylgruppe.

Durch 12-stündige Polymerisation des obigen Trisimids bei 250 °C erhält man einem Festkörper mit einer Glasumwandlungstemperatur über 300 °C.

Beispiel 9:

Herstellung des N,N' ,N"-Tris-(allyl-bicyclo[2.2.1]-hept-5-en-2,3-dicarbonsäureimids) des Tris(2-aminoethyl)phosphats. Allyl-bicyclo[2.2.1]hept-5-en-2,3-dicarbonsäure-N-(2'-hydroxyethyl)imid wird durch azeotrope Entwässerung eines Reaktionsgemisches aus 51 g Allylnadic-anhydrid 15,3 g 2-Amino-ethanol und 150 g Touol hergestellt. Nach dem Abdestillieren des Lösungsmittels verbleiben 61 g eines farblosen, viskosen Oels. Man löst 55,1 g dieses Zwischenproduktes in 200 g Toluol, setzt 24,8 g Triethylamin zu und tropft unter Rühren und äusserer Kühlung 11,3 g Phosphoroxychlorid so zu, dass die Temperatur zwischen 0 und 5 °C verbleibt. Man rührt über Nacht bei Raumtemperatur, neutralisiert mit 1 N HCl (pH 6,5), wäscht mit Wasser, trocknet die Toluolphase über $Na_2SO_4$, filtriert und destilliert das Lösungsmittel im Vakuum am Rotationsdampfer bei 90 °C ab. Es verbleiben 52,5 g (89,9 % der Theorie) eines gelbbraunen Harzes, das bei Raumtemperatur gerade noch flüssig ist. $\eta_{80}$: 3407 mPas.

| Elementaranalyse für $C_{42}H_{48}O_{10}N_3P$: | | | | |
|---|---|---|---|---|
| berechnet | C 64,19 % | H 6,16 % | N 5,35 % | P 3,94 % |
| gefunden | C 64,11 % | H 6,28 % | N 5,27 %. | P 3,86 % |

Anwendungsbeispiele I-V

Die gemäss den Beispielen 1, 3, 4 und 5 erhaltenen Trisimide werden direkt aus dem Reaktionsgemäss in Stahlformen von 12x12x0,4 cm gegossen, während je 3 Stunden bei 200 und 220 °C vorgeliert, während 12 Stunden bei 250 °C ausgehärtet und zu Prüfstäben zersägt. Analog werden 80 g eines 1:1-Gemisches des nach Beispiel 2 erhaltenen Trisimids und N,N'-Hexamethylen-bis(allyl-bicyclo[2.2.1]hept-5-en-2,3-dicarbonsäureimid) [hergestellt gemäss Beispiel 9 der EP-A 0 105 024] in Stahlformen gegossen, vorgeliert, ausgehärtet und zu Prüfstäben zersägt. Die Ergebnisse der Prüfung sind in der folgenden Tabelle zusammengefasst.

Anwendungsbeispiel VI:

80 g eines 1:1-Gemisches des nach Beispiel 1 erhaltenen Trismids und Allyl-bicyclo[2.2.1]hept-5-en-2,3-dicarbonsäure-N-allylimid (hergestellt gemäss Beispiel 3 der EP-A 0 105 024) wird als heisses Harz in eine Stahlform von 12x12x0,4 cm gegossen und wird während je 3 Stunden bei 200 °C und 220 °C und 12 Stunden bei 250 °C ausgehärtet.

Anwendungsbeispiel VII:

80 g eines 3:3:4-Gemisches des nach Beispiel 1 erhaltenen Trisimids; N,N'-4,4'-Diphenylmethanbismaleinimid und Allyl-bicyclo[2.2.1]hept-5-en-2,3-dicarbonsäure-N-allylimid wird als heisses Harz in eine Stahlform von 12x12x0,4 cm gegossen und während je 3 Stunden bei 200° C und 220° C und 12 Stunden bei 250° C ausgehärtet.

| | VI | VII |
|---|---|---|
| Biegefestigkeit ($N/mm^2$) | 63,5 | 78,1 |
| Randfaserdehnung (%) | 1,9 | 2,3 |
| Schlabiegefestigkeit ($hJ/m^2$) | 4,0 | 5,0 |
| Tg (°C) | 284 | 321 |

Tabelle

| Anwendungsbeispiel<br>Harz nach Beispiel | I<br>1 | II<br>3 | III<br>4 | IV<br>5 | V<br>2* | VI<br>1 | VII<br>1 |
|---|---|---|---|---|---|---|---|
| Biegefestigkeit nach DIN 53452 $N/mm^2$ | 91,6 | nicht giessbar | nicht giessbar | 97,2 | 98,2 | 63,5 | 78,1 |
| Randfaserdehnung nach ISO 178 (%) | 3,1 | - | - | 4,8 | 3,8 | 1,9 | 2,3 |
| Schlagbiegefestigkeit nach DIN 53455 ($kJ/m^2$) | 8,6 | - | - | 8,4 | 11,3 | 4,0 | 5,0 |
| Tg des Harzes (°C) | 311 | 250 | 310 | 150 | 279 | 284 | 321 |
| Zersetzungsbeginn des Harzes (°C) | 340 | 250 | 330 | n.b. | n.b. | | |
| 10 % Gewichtsverlust bei °C | 390 | 389 | 439 | 397 | n.b. | | |
| Brennbarkeit nach UL 94, Stufe | 0 | 0 | 0 | 1 | 0 | | |
| Wasseraufnahme nach 1 h bei 100°C (%) | 0,64 | n.b. | n.b. | 0,76 | 0,59 | | |

*) 1:1-Gemisch mit N,N'-Hexamethylen-bis(allyl-bicyclo[2.2.1]hept-5-en-2,3-dicarbonsäureimid).
n.b. = nicht bestimmt

## Ansprüche

1. Verbindungen der Formel I

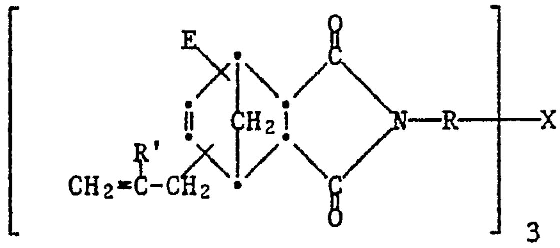 (I),

worin E und R' unabhängig voneinander ein Wasserstoffatom oder Methyl bedeuten,

X 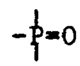 oder 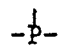

und R $-C_mH_{2m}-$ mit m = 2-20, $-C_nH_{2n}-O-$ mit n = 2-6, meta- oder para-Phenylen, meta- oder para-Phenylenoxy darstellen, wobei das Sauerstoffatom an die Gruppe X gebunden ist, oder

X 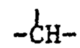

und R meta- oder para-Phenylen darstellen, oder X $CH_3CH_2C(CH_2-)_3$ oder

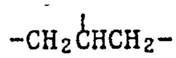

ist und R $-(CH_2CH_2O)_q-$, $-(CH_2CH_2CH_2O)_q-$ oder $-[CH_2CH(CH_3)O]_q-$ mit q unabhängig voneinander je 1-6, meta- oder para-Phenylenoxy darstellt, wobei das Sauerstoffatom an die Gruppe X gebunden ist, oder

X 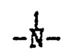

und R $-C_rH_{2r}-$ mit r = 2-4, meta- oder para-Phenylen darstellen.

**2.** Verbindungen der Formel I nach Anspruch 1, worin E und R' je ein Wasserstoffatom darstellen.

**3.** Verbindungen der Formel I nach Anspruch 1, worin

X 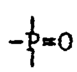 oder

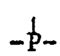

darstellt und R $-(CH_2)_m-$ mit m = 2-4, meta- oder para-Phenylen, meta- oder para-Phenylenoxy bedeutet.

**4.** Verbindungen der Formel I nach Anspruch 1, worin

X 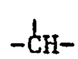

ist und R para-Phenylen darstellt.

**5.** Verbindungen der Formel I nach Anspruch 1, worin X $CH_3CH_2C(CH_2-)_3$ ist und die R -$[CH_2CH(CH_3)O]$-

$_q$- mit q = durchschnittlich 1,2 - 3 darstellen.

**6.** Verbindungen der Formel I nach Anspruch 1, worin

$$X\ -CH_2CHCH_2-$$

ist und R $-(CH_2CH_2O)_q-$ mit q = durchschnittlich 1,2-3 und insbesondere meta- oder para-Phenylenoxy darstellt.

**7.** Verbindungen der Forme I nach Anspruch 1, worin

$$X\ -N-$$

ist und R $-(CH_2)_r-$ mit r = 2 oder 3, meta- oder para-Phenylen darstellt.

**8.** Verbindungen gemäss Anspruch 1 der Formel I, worin E und R' Wasserstoff bedeuten und

$$X\ -P{=}O \text{ oder } -P-$$

ist und R para-Phenylenoxy bedeutet.

**9.** Das N,N' ,N"-Tris(allyl-bicyclo[2.2.1]hept-5-en-dicarbonsäureimid) des Polyoxypropylentriamins gemäss Anspruch 1.

**10.** Verfahren zur Herstellung von Verbindungen der Formel I nach Anspruch 1, dadurch gekennzeichnet, dass man eine Verbindung der Formel II

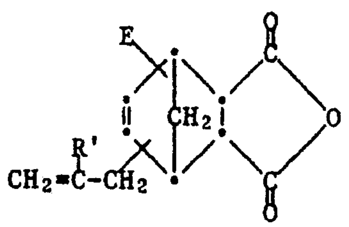

(II),

bei erhöhter Temperatur und unter Abdestillieren des bei der Reaktion entstehenden Wassers mit einer Verbindung der Formel III

$[H_2N-R]_3-X$ (III)

umsetzt, wobei für E, R, R' und X das im Anspruch 1 Angegebene gilt.

**11.** Verfahren zur Herstellung von Verbindungen der Formel I nach Anspruch 1, worin

$$X\ -P{=}O \text{ oder } -P-$$

und R $-C_nH_{2n}-O-$, meta- oder para-Phenylenoxy darstellen, dadurch gekennzeichnet, dass man eine Verbindung der Formel IV

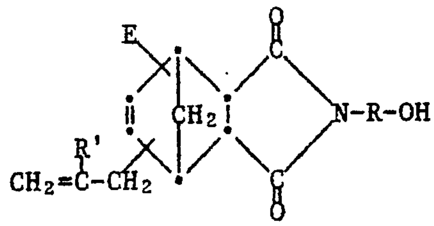

(IV),

worin E und R' die unter Formel I angegebene Bedeutung haben und R $-C_nH_{2n}$, meta- oder para-Phenylen darstellt, in Gegenwart einer Base im Molverhältnis von mindestens 1:3 mit einem Phosphoroxyhalogenid oder einem Phosphortrihalogenid umsetzt.

**12.** Polymere, die dadurch erhältlich sind, dass man mindestens eine Verbindung der Formel I nach Anspruch 1 auf Temperaturen zwischen 180 und 300 °C erhitzt.

**Claims**

**1.** A compound of formula I

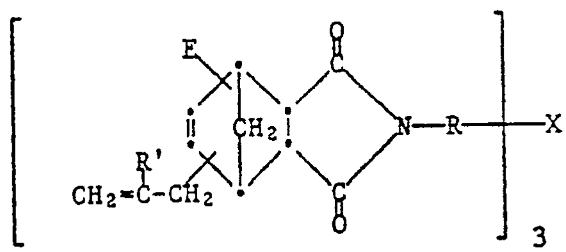

(I)

in which
E and R' are each independently of one another a hydrogen atom or methyl

X is -P=O or -P-

and R is $-C_mH_{2m}-$, in which m is 2 to 20, $-C_nH_{2n}-O-$, in which n is 2 to 6, meta or para-phenylene, meta or paraphenyleneoxy, in which the oxygen atom is attached to the group X, or

X is -CH-

and R is meta or para-phenylene, or X is $CH_3CH_2C(CH_2-)_3$ or

$-CH_2CHCH_2-$

and R is $-(CH_2CH_2O)_q-$, $-(CH_2CH_2CH_2O)_q-$ or $-[CH_2CH(CH_3)O]_q-$, in which q are each independently of one another 1 to 6, meta or paraphenyleneoxy, in which the oxygen atom is attached to the group X, or

X is -N-

and R is $-C_rH_{2r}-$, in which r is 2 to 4, or meta or para-phenylene.

**2.** A compound of formula I according to claim 1, in which each of E and R' is a hydrogen atom.

**3.** A compound of formula I according to claim 1, in which

$$X \text{ is } -\overset{|}{\underset{|}{P}}=O \text{ or}$$

$$-\overset{|}{P}-$$

and R is $-(CH_2)_m-$, in which m is 2 to 4, meta or para-phenylene or meta or para-phenyleneoxy.

**4.** A compound of formula I according to claim 1, in which

$$X \text{ is } -\overset{|}{C}H-$$

and R is para-phenylene.

**5.** A compound of formula I according to claim 1, in which X is $CH_3CH_2C(CH_2-)_3$ and each R is $-[CH_2CH(CH_3)O]_q-$, in which the value of q is on average from 1.2 to 3.

**6.** A compound of formula I according to claim 1, in which

$$X \text{ is } -CH_2\overset{|}{C}HCH_2-$$

and R is $-(CH_2CH_2O)_q-$, in which the value of q is on average from 1.2 to 3, and is in particular meta or para-phenyleneoxy.

**7.** A compound of formula I according to claim 1, in which

$$X \text{ is } -\overset{|}{N}-$$

and R is $-(CH_2)_r-$, in which r is 2 or 3, or is meta or para-phenylene.

**8.** A compound according to claim 1 of formula I, in which E and R' are hydrogen,

$$X \text{ is } -\overset{|}{\underset{|}{P}}=O \text{ or } -\overset{|}{\underset{|}{P}}-$$

and R is para-phenyleneoxy.

**9.** The N,N'N"-tris(allylbicyclo[2.2.1]hept-5-enedicarboximide) of the polyoxypropylenetriamine according to claim 1.

**10.** A process for the preparation of a compound of formula I according to claim 1, which comprises reacting a compound of formula II

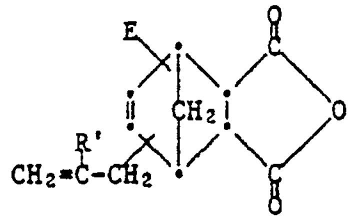

(II)

with a compound of formula III

$[H_2N\text{-}R]_3\text{-}X$ (III)

in which E, R, R' and X are as defined in claim 1, at elevated temperature and with removal of the water of reaction by distillation.

**11.** A process for the preparation of a compound of formula I according to claim 1, in which

$$X \text{ is } -\overset{|}{\underset{|}{P}}{=}O \text{ or } -\overset{|}{\underset{|}{P}}-$$

and R is $-C_nH_{2n}-O-$ or meta or para-phenyleneoxy, which comprises reacting a compound of formula IV

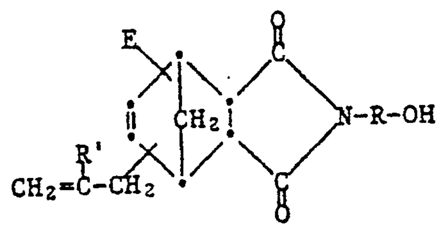

(IV)

in which E and R' are as defined for formula I and R is $-C_nH_{2n}-$ or meta or para-phenylene, with a phosphoroxy halide or a phosphorus trihalide, in the presence of a base in the molar ratio of at least 1:3.

**12.** A polymer obtainable by heating at least one compound of formula I according to claim 1 to temperatures in the range from 180 to 300 °C.

## Revendications

**1.** Composés répondant à la formule I :

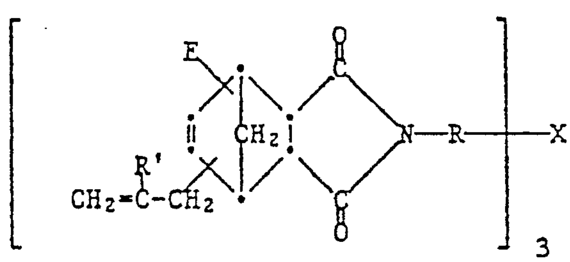

(I)

dans laquelle

E et R' représentent chacun, indépendamment l'un de l'autre, un atome d'hydrogène ou un radical méthyle,

X représente un radical

$$>P=O \text{ ou } -\overset{|}{P}-,$$

auquel cas R représente un radical $-C_mH_{2m}-$ dont l'indice m désigne un nombre de 2 à 20, un radical $-C_nH_{2n}-O-$ dont l'indice n représente un nombre de 2 à 6, un radical m- ou p-phénylène, ou un radical m- ou p-phénylène-oxy, l'atome d'oxygène étant lié au radical X, ou

X représente un radical

$$-\overset{|}{C}H-,$$

auquel cas R représente un radical m- ou p-phénylène, ou

X représente un radical $CH_3CH_2C(CH_2-)_3$ ou

$$-CH_2\overset{|}{C}HCH_2-,$$

auquel cas R représente $-(CH_2CH_2O)q-$, $-(CH_2CH_2CH_2O)_q-$ ou $-[CH_2CH(CH_3)O]_q-$, radicaux dans lesquels les indices q sont égaux chacun, indépendamment les uns des autres, à un nombre de 1 à 6, ou représente un radical m- ou p-phénylène-oxy, l'atome d'oxygène étant lié au radical X, ou

X représente un radical

$$-\overset{|}{N}-,$$

auquel cas R représente un radical $-C_rH_{2r}-$ dont l'indice r désigne un nombre de 2 à 4, un radical m-phénylène ou un radical p-phénylène.

**2.** Composés de formule I selon la revendication 1 dans lesquels E et R' représentent chacun un atome d'hydrogène.

**3.** Composés de formule I selon la revendication 1 dans lesquels X représente un radical

$$>P=O \text{ ou } -\overset{|}{P}-,$$

et R représente un radical $-(CH_2)m-$ dont l'indice m désigne un nombre de 2 à 4, un radical m- ou p-phénylène, ou un radical m- ou p-phénylène-oxy.

**4.** Composés de formule I selon la revendication 1 dans lesquels X représente un radical

$$-\overset{|}{C}H-$$

et R un radical p- phénylène.

**5.** Composés de formule I selon la revendication 1 dans lesquels X représente un radical $CH_3CH_2C(CH_2-)_3$, et les R représentent des radicaux $-[CH_2CH(CH_3)O]_q-$ dont l'indice q a une valeur moyenne comprise entre 1,2 et 3.

**6.** Composés de formule I selon la revendication 1 dans lesquels X représente radical

$$-CH_2\overset{|}{C}HCH_2-$$

et les R représentent des radicaux $-(CH_2CH_2O)_q-$ dont l'indice q a une valeur moyenne comprise entre 1,2 et 3, ou représentent plus particulièrement des radicaux m- ou p-phénylène-oxy.

**7.** Composés de formule I selon la revendication 1 dans lesquels X représente un radical

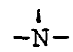

et R un radical $-(CH_2)_r-$ dont l'indice r est égal à 2 ou à 3, un radical m-phénylène ou un radical p-phénylène.

**8.** Composés de formule I selon la revendication 1 dans lesquels E et R' représentent chacun l'hydrogène, X représente

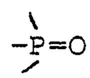 ou 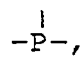,

et R un radical p-phénylène-oxy.

**9.** Composé selon la revendication 1, en l'espèce le N,N',N"-tris-(allyl-bicyclo[2.2.1]heptène-5 dicarboximide) de la poly-(oxy-propylène)-triamine.

**10.** Procédé pour préparer des composés de formule I selon la revendication 1, procédé caractérisé en ce qu'on fait réagir un composé de formule II :

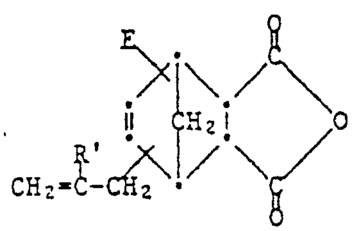

(II),

à température élevée et tout en chassant par distillation l'eau engendrée lors de la réaction, avec un composé de formule III :

$[H_2N-R]_3-X$ (III),

formules dans lesquelles E, R, R' et X ont les significations qui leur ont été données à la revendication 1.

**11.** Procédé pour préparer des composés de formule I selon la revendication 1 dans lesquels X représente un radical

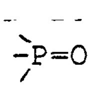 ou 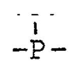

et R représente un radical $-C_nH_{2n}-O-$, m-phénylène-oxy ou p-phénylène-oxy, procédé caractérisé en ce qu'on fait réagir un composé répondant à la formule IV :

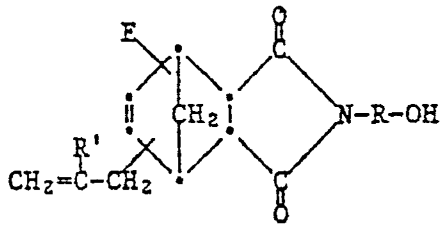

(IV)

dans laquelle E et R' ont les significations qui leur ont été données à propos de la formule I et R représente un radical $-C_nH_{2n}-$, m-phénylène ou p-phénylène, en présence d'une base, dans un rapport molaire d'au moins 1:3, avec un oxyhalogénure de phosphore ou un trihalogénure de phosphore.

**12.** Polymères qui peuvent être obtenus par chauffage d'au moins un composé de formule I selon la revendication 1 à des températures comprises entre 180 et 300 °C.